# EUROPÄISCHE PATENTSCHRIFT

(11) **EP 1 098 651 B1**
(45) Veröffentlichungstag und Bekanntmachung des Hinweises auf die Patenterteilung: **08.01.2003**
(21) Anmeldenummer: 99936570.3
(22) Anmeldetag: 20.07.1999
(51) Int. Cl.: A61K 31/495, A61P 35/00

(54) **NEUE UROKINASE-INHIBITOREN**
NOVEL UROKINASE INHIBITORS
NOUVEAUX INHIBITEURS D'UROKINASE

(30) Priorität: 20.07.1998 EP 98113519
(43) Veröffentlichungstag der Anmeldung: 16.05.2001
(60) Teilanmeldung: 02016038.8
(73) Patentinhaber: Wilex AG, 81675 München (DE)
(72) Erfinder: WILHELM, Olaf, D-81545 München (DE); MAGDOLEN, Viktor, D-85551 Kirchheim (DE); STÜRZEBECHER, Jörg, D-99094 Erfurt (DE); FOEKENS, John, NL-2908 XA Capelle a/d IJssel (NL); LUTZ, Verena, D-81669 München (DE)
(74) Vertreter: Weiss, Wolfgang, Dr. Dipl.-Chem.
(86) Internationale Anmeldenummer: EP9905145
(87) Internationale Veröffentlichungsnummer: WO00004954

(56) Entgegenhaltungen:
- EP-A- 0 183 271
- WO-A-92/08709
- WO-A-94/18185
- WO-A-95/17885
- WO-A-96/05189
- DE-A- 3 035 086

## Beschreibung

Die Erfindung betrifft die Verwendung von Derivaten des 3-Amidinophenylalanins als Urokinase-Inhibitoren insbesondere zur Behandlung von malignen Tumoren und der Metastasierung bzw. als Mittel zum Targeting von Lymphzellen und zur Behandlung von Erkrankungen des Lymphgewebes, insbesondere von Lymphomen.

Die Fähigkeit solider Tumoren zur Ausbreitung und Metastasierung in umgebendes Gewebe korreliert mit dem Abbau bzw. Umbau der extrazellulären Matrix (Tumorstroma) in der Umgebung der Tumorzelle, bzw. mit deren Fähigkeit zur Durchdringung der Basalmembran. Obwohl die (patho)biochemischen Zusammenhänge noch nicht endgültig aufgeklärt sind, kommen dem Plasminogenaktivator Urokinase (uPA) und dem Urokinaserezeptor (uPAR) eine zentrale Bedeutung zu. uPA vermittelt die proteolytische Spaltung von Plasminogen zu Plasmin. Plasmin wiederum ist eine Protease mit breitem Wirkspektrum, die Komponenten der extrazellulären Matrix wie Fibrin, Fibronektin, Laminin und das Proteingerüst der Proteoglykane direkt abzubauen vermag. Außerdem kann Plamin "latente" Metalloproteasen und das inaktive Proenzym von uPA, pro-uPA, aktivieren.

Tumorzellen und nichtmaligne Zellen des Tumorstromas synthetisieren und sezernieren das enzymatisch inaktive Proenzym pro-uPA. Proteasen wie z.B. Plasmin oder die Kathepsine B und L spalten pro-uPA durch limitierte Proteolyse zur aktiven Serinprotease HMW-uPA (HMW=high molecular weight). pro-uPA und die aktive Protease HMW-uPA binden an den Zelloberflächenrezeptor uPAR (CD87). Plasmin(ogen) bindet ebenfalls an spezifische Rezeptoren auf der Plasmamembran der Tumorzelle, wodurch eine Fokussierung und Amplifikation der Plasminogenaktivierung in der direkten Umgebung der Tumorzelle erreicht wird. Invasiven Zellen ist somit die Möglichkeit gegeben, die extrazelluläre Matrix abzubauen, ohne sich der für eine gerichtete Bewegung notwendigen Unterlagen durch Proteolyse zu entziehen.

In verschiedenen zellbiologischen Studien konnte gezeigt werden, daß dem zellassoziierten Plasminogenaktivator-System innerhalb der kaskadenartigen Reaktionswege tumorassoziierter Proteolysesyteme ein besonderer Stellenwert zukommt (Wilhelm et al. (1994) The Urokinase/Urokinase receptor system: A new target for cancer therapy? In: Schmitt M., Graeff H., Kindermann G. (Hrsg): Prospects in Diagnosis and Treatment of Cancer. International Congress Series, Excerpta Medica 1050, Amsterdam, Elsevier 1994, pp145-156). An Kulturen humaner Kolonkarzinomzellen wurde beobachtet, daß deren Fähigkeit, eine extrazelluläre Matrix zu durchwandern, vom Sättigungsgrad der uPA-Rezeptoren mit aktivem uPA abhängig ist (Hollas et al., Cancer Res. 51 (1991), 3690-3695). Ebenfalls im Zellkulturmodell wurde eine Reduktion des invasiven Potentials von Zellen beobachtet, wenn die proteolytische Aktivität von uPA durch PAI-1 (Cajot et al., Proc. Natl. Acad. Sci. USA 87 (1990), 6939-6943) oder PAI-2 (Baker et al., Cancer Res. 50 (1990), 4676-4684) gehemmt wurde. Ein vergleichbarer Effekt wurde bei Hemmung der Bindung von uPA an die Zelloberfläche durch Blockierung des Rezeptors mittels proteolytisch inaktiver uPA-Varianten erzielt (Cohen et al., Blood 78 (1991), 479-487; Kobayashi et al., Br. J. Cancer 67 (1993), 537-544). Auch die Transfektion epidermoider Karzinomzellen mit einem Plasmid, das ein Antisense-Transkript gegen einen Teil von uPAR exprimiert, führte durch Unterdrückung der uPAR-Synthese zur Verringerung der Invasivität dieser Zellen (Kook, EMBO J. 13 (1994), 3983-3991). Gegen uPA und PAI-1 gerichtete Antikörper reduzierten das invasive Potential von Lungenkrebszellen in vitro (Liu et al., Int. J. Cancer 60 (1995), 501-506).

Der Einfluß des Plasminogenaktivator-Systems auf den Metastasierungsprozeß konnte auch in Tumor-Tiermodellen belegt werden. So wurden die durch menschliche Karzinomzellen verursachte Bildung von Lungenmetastasen in Hühnerembryos durch Zugabe von Antikörpern gegen uPA fast vollständig verhindert (Ossowski und Reich, Cell 35 (1983), 611-619). Metastasierende menschliche Karzinomzellen wurden mit einem Expressionsplasmid transfiziert, das für eine proteolytisch inaktive, aber uPARbindende uPA-Mutante kodierte. Im Mausmodell zeigte sich, daß die Karzinomzellen, die inaktives uPA synthetisierten, nach Injektion im Vergleich zu den nichttransfizierten Zellen eine signifikant geringere Anzahl an Metastasen bildeten (Crowley et al., Proc. Natl. Acad. Sci. USA 90 (1993), 5021-5025). Nach Verabreichung von uPA-Antisense Oligonukleotiden wurde darüber hinaus eine Inhibierung der intraperitonealen Ausbreitung von humanen Ovarialkarzinomzellen in Nacktmäusen beobachtet (Wilhelm et al., Clin. Exp. Metast. 13 (1995). 296-302).

In den letzten Jahren wurde die klinische Relevanz von Faktoren des Plasminogenaktivator-Systems (uPA, uPAR, PAI-1 und PAI-2) für die Prognose von Patienten mit soliden malignen Tumoren intensiv untersucht. Dabei erwies sich der uPA-Antigengehalt bei verschiedenen Tumoren (z.B. Brust, Eierstock, Magen, Lunge, Niere etc.) sowohl für das rezidivfreie Überleben als auch für das Versterben als ein starker Prognosefaktor (siehe beispielsweise Schmitt et al.,-J. Obstet. Gynaecol. 21 (1995), 151-165; Jaenicke et al., Breast Cancer Res. Treat. 24 (1993), 195-208; Kuhn et al., Gynecol. 0ncol. 55 (1994), 401-409; Nekarda et al., Lancet 343 (1994), 117; Pedersen et al., Cancer Res. 54 (1994), 4671-4675). Ebenso korrelieren erhöhte Konzentrationen an uPAR in Lungen- (Pedersen et al., supra) und Brustkrebsgewebe (Duggan et al., Int. J. Cancer 61 (1995), 597-600; Ronne et al., Breast Cancer Res. Treat. 33 (1995), 199-207) sowie bei Magenkrebs sowohl im Tumorgewebe selbst (Heiss et al., J. Clin. Oncol. 13 (1995), 2084-2093) als auch bei den ins Knochenmark ausgestreuten Tumorzellen (Heiss et al., Nature Medicine 1 (1995), 1035-1039) mit einer schlechten Prognose.

Der Einsatz von sythetischen uPA-Inhibitoren bietet die Möglichkeit, die Invasion und Ausbreitung von Tumorzellen zu unterdrücken. Allerdings ist die Entwicklung spezifischer uPA-Inhibitoren mit Schwierigkeiten behaftet, da der Plasminogenaktivator von Gewebetyp (tPA) eine identische Spezifität für die Spaltung der Peptidbindung Arg560/Val561 von Plasminogen aufweist. In den meisten Fällen hemmen daher niedermolekulare uPA-Inhibitoren auch tPA und damit auch tPA-vermittelte Fibrinolyse. Außerdem muß gewährleistet sein, daß synthetische uPA Inhibitoren keine starke Hemmung von Plasmin zeigen.

Trotz dieser Einschränkungen sind einige Hemmstoffe bekannt, die eine gewisse Spezifität gegenüber uPA, jedoch geringe inhibitorische Kapazität besitzen, wie etwa Benzamidin- und β-Naphthamidin-Derivate, wobei die wirksamste Verbindung uPA mit Kᵢ=2,2 µmol/l hemmt (Stürzebecher und Markwardt, Pharmazie 33 (1978), 599), oder Amilorid mit einem Kᵢ=7 µmol/l (Vassalli und Belin, FEBS. Lett. 214 (1987), 187-191).

DE-A-30 35 086 offenbart Cyclohexancarbonsäurederivate, die inhibitorische Wirkungen auf Proteasen wie Trypsin, Chymotrypsin, Thrombin oder uPA haben. Die untersuchten Verbindungen zeigen jedoch nur eine recht geringe und darüber hinaus unspezifische uPA-inhibierung. EP-A-0 183 271 offenbart Lysinderivate und deren Verwendung als Proteaseinhibitoren. Es wird auch ein Benzamidino-Lysinderivat (Verbindung 108) beschrieben, das in vitro eine uPA-Hemmung, jedoch auch eine vergleichbare Wirkung auf andere Proteasen wie Trypsin oder Plasma-Kallikrein aufweist. WO 95/17885 offenbart niedermolekulare Polypeptide als uPA-Inhibitoren.

Eine weitere Klasse von bekannten uPA-inhibitoren stellen 4-substituierte Benzothiophen-2-carboxamidine mit einem Kᵢ=0,16 mmol/l im Falle von Benzothiophen-623 dar (Towle et al., Cancer Res. 53 (1993), 2553-2559). Diese Hemmstoffe haben eine signifikant höhere Affinität für uPA im Vergleich zu tPA und Plasmin. Auch uPAR-gebundenes uPA wird mit hoher Effizienz gehemmt. Ein Nachteil dieser Substanzen liegt allerdings darin, daß die chemische Synthese kompliziert ist und kaum Möglichkeiten für die Modifizierung der Struktur vorhanden sind, bzw. bisher gezeigt werden konnte.

Die Entwicklung weiterer Hemmstoffe von uPA ist daher für die weitere Aufklärung der Rolle von uPÄ und uPAR bei verschiedenen Krankheiten, speziell bei der Tumorausbreitung und Metastasierung, von großem Nutzen.

Nα-Arylsulfonyl- und Nα-Arylsulfonyl-amino-acyl-Derivate des 3-Amidinophenylalanins sind als selektive Hemmstoffe von Thrombin (Markwardt et al., Thromb. Res. 17 (1980), 425-431) bzw. von Gerinnungsfaktor Xa (Stürzebecher et al., Thromb. Res. 54 (1989), 245-252) bekannt. Auch in WO 92/08709, WO 94/18185 und WO 96/05189 wird die Verwendung von Amidinophenylalaninderivaten als Hemmstoffe für die Blutgerinnung, insbesondere als Hemmstoffe für Thrombin, offenbart.

Intensiv wurden Piperidide und Piperazide des 3-Amidinophenylalanins untersucht, unter denen auch Leitstrukturen zur Hemmung fibrinolytischer Enzyme gefunden wurden (Stürzebecher et al., J. Enzyme Inhibition 9,87-99, 1995; Stürzebecher et al., J. Med. Chem. 40, 3091-3099, 1997). Während bei Stürzebecher et al. (1995) nur eine Hemmung von Thrombin, Faktor Xa, Plasmin und Trypsin beschrieben ist, finden sich bei Stürzebecher et al. (1997) auch Angaben zur Hemmung von uPA. Bei Nα-2-Naphthylsulfonyl-, Nα-2-(2,2,5,7,8-Pentamethylchroman-6-yl)sulfonyl-und Nα-2-Campher-10-yl-sulfonyl-substituierten 3-Amidinophenylalaninpiperaziden wird für uPA ein Kᵢ-Wert von 28 bis 140 µmol/l gefunden, der um drei Größenordnungen höher als die Inhibitionskonstante für Thrombin ist. Somit konnte nicht davon ausgegangen werden, daß 3-Amidinophenylalaninderivate als Urokinaseinhibitoren geeignet sind.

Überraschenderweise wurde jedoch gefunden, daß an Position 2 mit einem Phenylrest substituierte 3-Amidinophenylalaninderivate selektive und in vivo wirksame Hemmstoffe von uPA darstellen. Weiterhin wurde gefunden, daß diese Substanzen eine hohe Selektivität für Lymphgewebe aufweisen und sich daher als Mittel zum Targeting von Lymphzellen, beispielsweise zur Behandlung von malignen Erkrankungen des Lymphgewebes wie etwa Lymphomen eignen.

Die vorliegende Erfindung betrifft von 3-Amidinphenylalanin abgeleitete neue Urokinase-Inhibitoren der allgemeinen Formel I, 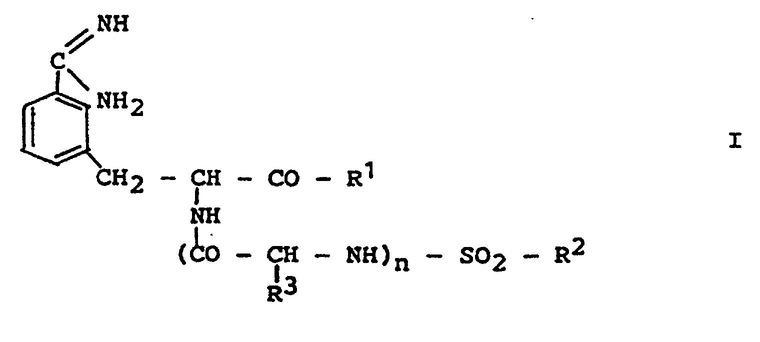 die als Racemate sowie als L- bzw. D-konfigurierte Verbindungen vorliegen und in denen
- R1: (a) OH oder OR⁴ ist, wobei R⁴ ein gegebenenfalls z.B. mit Hydroxyl, Carboxyl, Sulfonyl, Nitro, Cyano, Oxo oder/und Halogen substituiertes, verzweigtes oder unverzweigtes C₁-C₈-Alkyl, C₃-C₈-Cycloalkyl oder Aralkyl, z.B. Benzyl oder Phenylethyl ist,
(b) eine Gruppe der Formel 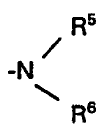 darstellt, in welcher R⁵ und R⁶ beliebige mit der Gesamtstruktur kompatible Reste sind, wobei insbesondere
   (i) R⁵ und R⁶ H sind,
   (ii) R⁵ H ist und R⁶ ein gegebenenfalls z.B. mit Hydroxyl, Carboxyl, Sulfonyl, Nitro, Cyano, Oxo oder/und Halogen substituiertes verzweigtes oder unverzweigtes C₁-C₈-Alkyl, Aralkyl, z.B. Benzyl oder Phenylethyl, oder C₅-C₈ Cycloalkyl ist,
   (iii) R⁵ und R⁶ jeweils unabhängig ein gegebenenfalls z.B. mit Hydroxyl oder/und Halogen substituiertes, unverzweigtes oder verzweigtes C₁-C₄ Alkyl sind oder
   (iv) R⁵ H ist und R⁶ -NH₂ oder eine insbesondere mit Aryl oder Heteroaryl substituierte Aminogruppe ist,
   (v) R⁵ H oder ein gegebenenfalls z.B. mit Hydroxyl oder/und Halogen substituiertes, unverzweigtes oder verzweigtes C₁-C₄ Alkyl ist und R⁶ der Rest einer Aminosäure, z.B. einer α-, β- oder ω-Aminocarbon- oder Aminosulfonsäure, oder der Rest eines Peptids z.B. mit einer Länge bis zu 50 Aminosäuren oder eines Polypeptids z.B. mit einer Länge von mehr als 50 Aminosäuren bis 1000 Aminosäuren ist,
(c) eine Gruppe der Formel 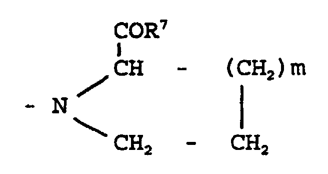 darstellt, in welcher m die Zahl 1 oder 2 bezeichnet, und in welcher eine oder mehrere der Methylengruppen gegebenenfalls z.B. mit einem Hydroxyl-, Carboxyl-, C₁-C₄-Alkyl- oder Aralkylrest, z.B. Benzyl oder Phenylethyl, substituiert sind, wobei die Gruppe (c) racemisch oder D- bzw. L-konfiguriert ist, und R⁷ die Bedeutung von R¹ in den Ziffern (a), (b) und (f) aufweist,
(d) eine Gruppe der Formel 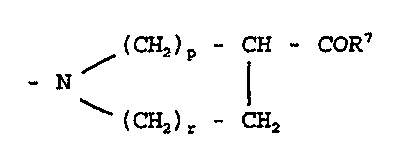 darstellt, in welcher p = r = 1, p = 1 und r = 2 oder p = 2 und r = 1 sind und in welcher eine oder mehrere der Methylengruppen gegebenenfalls z.B. mit einem Hydroxyl-, Carboxyl-, C₁-C₄-Alkyl- oder Aralkylrest, z.B. Benzyl oder Phenylethyl, substitutiert sind, und R⁷ die Bedeutung von R¹ in Ziffer (a), (b) und (f) aufweist,
(e) eine Piperidylgruppe darstellt, die gegebenenfalls in einer der Stellungen 2, 3 und 4 z.B. mit einem C₁-C₄-Alkyl- C₁-C₃-Alkoxy- oder Hydroxylrest substituiert ist,
   wobei gegebenenfalls an die heterocycloaliphatischen Ringe der Formeln (c), (d), (e) ein weiterer aromatischer oder cycloaliphatischer Ring, vorzugsweise Phenyl oder Cyclohexyl, in 2,3 oder 3,4 Stellung, bezogen auf das Heteroatom, ankondensiert ist,
(f) eine Gruppe der Formel 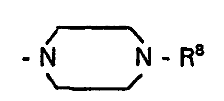 darstellt, in welcher R⁸
   (i) einen gegebenenfalls z.B. mit C₁-C₆-Alkyl, C₁-C₃-Alkoxy, Hydroxyl, Carboxyl, Sulfonyl, Nitro, Cyano, Oxo oder/und Halogen substituierten C₁-C₆-Alkyl- oder Arylrest, wie z.B. Phenyl, p-Halogenphenyl, Naphthyl,
   (ii) einen gesättigten oder ungesättigten, verzweigten oder unverzweigten C₁-C₆-Alkoxyrest oder
   (iii) einen gegebenenfalls z.B. mit C₁-C₆-Alkyl, C₁-C₃-Alkoxy, Hydroxyl, Carboxyl, Sulfonyl, Nitro, Cyano, Oxo oder/und Halogen substituierten Phenoxy- bzw. Benzyloxycarbonylrest bedeutet,
(g) einen Acylrest der Formel -COX darstellt, wobei X
   (i) H, einen gegebenenfalls z.B. mit Hydroxyl, Carboxyl, Sulfonyl, Nitro, Cyano, Oxo oder/und Halogen substituierten, unverzweigten oder verzweigten Alkylrest, vorzugsweise einen C₁-C₆-Alkylrest, insbesondere Methyl,
   (ii) einen gegebenenfalls z.B. mit C₁-C₆-Alkyl, C₁-C₃-Alkoxy, Hydroxyl, Carboxyl, Sulfonyl, Nitro, Cyano, Oxo oder/und Halogen substituierten Aryl- oder Heteroarylrest, wie z.B. Phenyl, p-Halogenphenyl, Thienyl oder
   (iii) einen gegebenenfalls z.B. mit Hydroxyl, Carboxyl, Sulfonyl, Nitro, Cyano, Oxo oder/und Halogen substituierten Cycloalkylrest, vorzugsweise einen C₃-C₁₀-Cycloalkylrest bedeutet,
(h) einen Aralkylrest, z.B. Benzyl oder Phenylethyl, darstellt, in dem der aromatische Rest gegebenenfalls z.B. mit einem Halogenatom, einer C₁-C₆-Alkyl-, C₁-C₃-Alkoxy-, Hydroxy-, Cyano-, Carboxyl.- Sulfonyl- oder Nitrogruppe substituiert ist,

(i) einen Carbonsäureamidrest der Formel -CONR'R", einen Thiocarbonsäureamidrest -CSNR'R" oder einen Essigsäureamidrest -CH₂-CONR'R" darstellt, wobei
   (i) R' und R" H sind,
   (ii) R' und R" jeweils unabhängig C₁-C₄-Alkyl sind,
   (iii) R' H ist und R" C₁-C₄-Alkyl ist,
   (iv) R' H ist und R" Aryl, z.B. Phenyl, ist oder
   (v) R' und R" mit dem Stickstoffatom einen heterocycloaliphatischen Ring mit 5-7 Ringgliedern, der ein weitere Heteroatom z.B. N, O oder/und S tragen kann, bilden,
(j) einen SO₂-Y-Rest darstellt, in dem Y
   (i) ein gegebenenfalls z.B. mit Hydroxyl, Carboxyl, Sulfonyl, Nitro, Cyano, Oxo oder/und Halogen substitutiertes C₁-C₈-Alkyl, vorzugsweise Methyl, Trifluormethyl, Trichlormethyl,
   (ii) ein gegebenenfalls z.B. mit C₁-C₆-Alkyl, C₁-C₃-Alkoxy, Hydroxyl, Carboxyl, Sulfonyl, Nitro, Cyano, Oxo oder/und Halogen substituiertes Aryl oder Heteroaryl, wie z.B. Phenyl, 4-Methyl-phenyl, 2,4,6-Trimethylphenyl, 2,4,6-Triisopropyl-phenyl, 4-Methoxy-2,3,6-Trimethyl-phenyl, 2,2-Dimethyl-6-methoxy- chromanyl, 2,2,5,7,8-Pentamethyl-chromanyl, Anthrachinonyl, Naphthyl oder Chinolyl, bzw. O-Aryl, vorzugsweise O-Phenyl, oder O-Heteroaryl oder
   (iii) -NR'R" ist, wobei R' und R" jeweils unabhängig H oder C₁-C₃-Alkyl bedeuten,
(k) einen cycloaliphatischen Ring mit 5 bis 8 C-Atomen darstellt, der ggf. z.B. mit einer C₁-C₆-Alkyl-, C₁-C₃-Alkoxy-, Halogen-, Hydroxyl- oder/und Oxogruppe substituiert ist,
(I) einen gegebenenfalls z.B. mit C₁-C₆-Alkyl, C₁-C₃-Alkoxy, Hydroxyl. Carboxyl, Sulfonyl, Nitro, Cyano, Oxo oder/und Halogen substituierten Heteroarylrest, wie z.B. Pyridyl oder Pyrimidyl, oder heterocycloaliphatischen Rest, beispielsweise N-Methylpiperidyl darstellt,
(m) einen funktionalisierten Alkylrest der Formel -(CH₂)ₙ-X darstellt, wobei die Alkylkette unverzweigt oder verzweigt ist, n = 1 bis 8 bedeutet und der funktionelle Rest X
   (i) eine Hydroxylgruppe darstellt, deren H-Atom gegebenenfalls durch eine C₁-C₄-Alkyl-, Aralkyl-, z.B. Benzyl oder Phenylethyl, Aryl-, z.B. Phenyl, C₁-C₄-Hydroxyalkyl- oder Acylgruppe CO-Alkyl, (C₁-C₆), substituiert ist,
   (ii) ein Halogenatom bedeutet,
   (iii) eine tertiäre Aminogruppe der Formel -N(Alk)₂ darstellt,
   wobei die Alkylgruppen 1 bis 3 C-Atome sowie vorzugsweise die gleiche Bedeutung besitzen und das Stickstoffatom gegebenenfalls einem heterocycloaliphatischen Ring mit 5-7 Ringgliedern, der ein weiteres Heteroatom, z.B. N, O oder/und S tragen kann, angehört.
- R²: einen gegebenenfalls z.B. mit C₁-C₆-Alkyl, C₁-C₃-Alkoxy, Hydroxyl, Carboxyl, Sulfonyl, Nitro, Cyano, Oxo oder/und Halogen substituierten Phenylrest, wie beispielsweise Phenyl, 4-Methyl-phenyl, 2,4,6-Trimethyl-phenyl, 2,4,6-Triisopropyl-phenyl, 4-Methoxy-2,3,6-trimethyl-phenyl darstellt,
- R³: H oder verzweigtes bzw. unverzweigtes C₁-C₄-Alkyl ist und n 0 oder 1 bedeutet.

Die Verbindungen können auch als Salze vorzugsweise als physiologisch verträgliche Säuresalze, z.B. als Salze von Mineralsäuren, besonders bevorzugt als Hydrochloride, oder als Salze von geeigneten organischen Säuren vorliegen.

Von den in den allgemeinen Ansprüchen definierten Verbindungen sind solche, bei denen R¹ einer Gruppe der Formeln (b), (d) und (f) entspricht, R² einen einfach, zweifach oder dreifach alkylsubstituierten Phenylrest, insbesondere einen 2,4,6-substituierten Phenylrest, z.B. einen 2,4,6-Triisopropylphenyl-Rest darstellt und n = 0 ist, von besonderer Bedeutung.

Die Verbindungen der allgemeinen Formel I können in prinzipiell bekannter Weise, wie z.B. in WO 92/08709 und WO 94/18185 beschrieben, hergestellt und auf ihre biologische in vitro Aktivität gesetzt werden.

(L)-, (D)- oder (D,L)-3-Cyanphenylalanin-methylester-hydrochlorid wird mit einem entsprechenden Sulfochlorid oder einer sulfonylierten Aminosäure bzw. deren Halogenid in Gegenwart einer Base zu einer Verbindung der allgemeinen Formel I mit Cyanfunktion, in der R¹ = OCH₃ ist und R² sowie R³ und n den in den allgemeinen Ansprüchen definierten Bedeutungen entspricht, umgesetzt. Durch milde saure oder alkalische Hydrolyse sind daraus die Verbindungen der allgemeinen Formel I mit Carbonsäurestruktur (R¹ = -OH) erhältlich, deren Veresterung mit einem entsprechenden Alkohol unter säurekatalytischen Bedingungen zu Verbindungen der allgemeinen Formel I führt, wobei R¹ = (a) bedeutet. Nach einem in der Peptidchemie üblichen Verfahren, z.B. DCC-Verfahren in Gegenwart von HOBt, sind durch Umsetzung der Carbonsäuren der allgemeinen Formel I (R¹ = -OH) mit einem Nukleophil der Strukturen (b), (e) und (f) die Verbindungen mit entsprechendem R¹ der allgemeinen Formel I vorstellbar. Für die Synthese von Verbindungen mit R¹ = (c) und (d) werden zunächst die Carbonsäuren der allgemeinen Formel I mit R¹ = OH mit cycloaliphatischen Aminosäureestern der Strukturen (c) und (d), wobei R⁷ vorzugsweise -OCH₃ bzw. -OC₂H₅ bedeutet, umgesetzt, die erhaltenen Carbonsäurester unter milden sauren oder alkalischen Bedingungen zu den entsprechenden Carbonsäuren hydrolysiert, die nachfolgend in bereits beschriebener Weise verestert oder mit Nukleophilen der Struktur (b), (e) und (f) umgesetzt werden können, wobei Verbindungen der allgemeinen Formel I mit R¹ = (c) sowie (d) und R⁷ = (a), (b), (e) und (f) erhalten werden.

Die Zielverbindungen der allgemeinen Formel I mit Amidinstruktur sind aus den Cyanverbindungen in bekannter Weise erhältlich, wobei in der Regel durch Addition von H₂S an die Cyangruppe zunächst die Thioamide erhalten werden, die durch S-Methylierung mit Methyliodid in die Thioimidoester und anschließend durch Behandlung mit Ammoniumacetat in alkoholischer Lösung in die Amidinoverbindungen überführt werden. Außerdem können gegebenenfalls aus den Cyanverbindungen mit Methanol oder Ethanol in Gegenwart von HCI-Gas und in bestimmten Fällen eines inerten Lösungsmittels die entsprechenden Imidoesterhydrochloride dargestellt werden, deren Umsetzung in alkoholischer Ammoniaklösung zu den Amidinoverbindungen führt.

Die erfindungsgemäßen Urokinaseinhibitoren können gegebenenfalls zusammen mit mindestens einem geeigneten pharmazeutischen Hilfs- oder Trägerstoff zur Herstellung von oral, subkutan oder intravenös verabreichbaren Arzneimitteln zur Tumorbekämpfung oder in der Diagnostik verwendet werden. Ebenfalls möglich ist die Verabreichung in Kombination mit anderen Wirkstoffen, z.B. anderen Urokinaseinhibitoren wie etwa Antikörpern oder/und Peptiden.

Die Arzneimittel zur Tumorbekämpfung bei Menschen und Tieren können topisch, oral, rektal oder parenteral, z.B. subkutan oder intravenös, in Form von Tabletten, Dragees, Kapseln, Pellets, Suppositorien, Lösungen oder transdermalen Systemen, wie Pflastern, verabreicht werden.

Besonders bevorzugt handelt es sich bei der Verbindung der Formel (I) um Nα-(2,4,6-Triisopropyl-phenylsulfonyl)-3-amidino-(D,L)-phenylalanin-4-ethoxycarbonyl-piperazid-hydrochlorid bzw. um das L-Entantiomere davon oder um ein pharmazeutisch verträgliches Salz dieser Verbindungen. Diese Substanzen haben ein gutes Löslichkeitsverhalten. In Trispuffer (pH 7,3) sind sie bis zu einer Konzentration von 5 x 10⁻⁵ mol/l löslich. Durch Zusatz von 5% Ethanol steigt die Löslichkeit auf 2x10⁻⁴mol/l und bei Zusatz von 5% DMSO auf 10⁻³ mol/l.

Die erfindungsgemäßen Verbindungen sind in der Lage, hocheffizient das Wachstum oder/und die Ausbreitung von malignen Tumoren zu hemmen, z.B. die Tumorausbreitung beim Pankreaskarzinom, das Tumorwachstum des Mammakarzinoms sowie die Metastasierung von Tumoren. Dabei können die uPA-Inhibitoren gegebenenfalls zusammen mit anderen Antitumormitteln oder mit anderen Behandlungsarten, z.B. Bestrahlung oder chirurgischen Eingriffen, eingesetzt werden. Weiterhin sind die erfindungsgemäßen Inhibitoren auch für andere uPA-assoziierte Erkrankungen wirksam (z.B. bei der Verhinderung der Blasenbildung bei der Hauterkrankung Pemphigus vulgaris).

Erfindungsgemäße uPA Inhibitoren sind vorzugsweise dadurch gekennzeichnet, daß sie einen mindestens zweifach, vorzugsweise mindestens 5-fach und besonders bevorzugt mindestens 10-fach geringeren Kᵢ-Wert für uPA gegenüber tPA aufweisen. Weiterhin ist bemerkenswert, daß die erfindungsgemäßen Verbindungen die Blutgerinnung nur geringfügig beeinflussen, da sie für eine effektive Hemmung von Thrombin und Faktor Xa zu hohe Kᵢ-Werte-haben.

Die erfindungsgemäßen Substanzen der Formel I können in Form von Konjugaten mit physiologisch wirksamen Substanzen eingesetzt werden, z.B. mit Radiomarkierungen oder mit zytotoxischen Mitteln, z.B. Chemotherapeutika wie cis-Platin oder 5-Fluor-Uracil, oder Peptiden. Weiterhin können die Substanzen auch in die Membran von Trägervesikeln, z.B. Liposomen, eingebaut werden und somit ein Targeting von in den Trägervesikeln eingeschlossenen Wirksubstanzen, z.B. zytotoxischen Mitteln wie etwa Doxorubicin ermöglichen.

Die Erfindung soll an den folgenden Beispielen und Abbildungen näher erläutert werden. Es zeigen:
- Figur 1: das Ergebnis einer Zytotoxizitätsbestimmung einer erfindungsgemäßen Substanz,
- Figur 2: das Ergebnis eines Versuchs zur Inhibierung des Abbaus einer Fibrinmatrix durch humane Mammakarzinomzellen,
- Figuren 3 und 4: die Wirkung einer erfindungsgemäßen Substanz auf die Ausbreitung, das Wachstum und die Metastasierung von Mammakarzinomzellen in der Ratte,
- Figur 5: die Wirkung einer erfindungsgemäßen Substanz auf das Wachstums eine Pankreastumors in der Ratte und
- Figur 6: die Wirkung einer erfindungsgemäßen Substanz auf das Wachstum humaner Mammakarzinomzellen in Mäusen.

### Beispiele

### 1. Nα-2,4,6-Triisopropylphenylsulfonyl-(L)-3-amidino-phenylalanin-4-ethoxycarbonyl-piperazid-hydrochlorid

### 1.1 Nα-2,4,6-Triisopropylphenylsulfonyl-(L)-3-cyan-phenylalanin-methylester

5 g (L)-3-Cyanphenylalanin-methylester wurden in 100 ml Dioxan suspendiert, 4;45 ml N-Methylmorpholin (NMM) zugefügt und 30 min gerührt. Nach Zugabe von 5,97 g 2,4,6-Triisopropylbenzolsulfochlorid in fester Form wurde 3 Tage gerührt, danach ausgefallenes NMM-HCI abfiltriert, das Lösungsmittel abdestilliert und das erhaltene Rohprodukt über Kieselgel (KG) 60 (Chloroform) gereinigt. Ausbeute: 8,34 g Sirup (90%).

### 1.2 Nα-2,4,6-Triisopropylphenylsulfonyl-(L)-3-cyan-phenylalanin

8,34 g der Verbindung 1.1 wurden in einer Mischung aus je 50 ml Essigsäure und 1 N Salzsäure 8 Std. unter Rückfluß erhitzt, nach dem Erkalten 2x mit Ethylacetat extrahiert, die vereinigten Ethylacetatlösungen über MgSO₄ getrocknet und das Lösungsmittel abdestilliert. Nach Reinigung über KG 60 (Chloroform) wurden 5,8 g eines festen Produktes erhalten (72%).

### 1.3 Nα-2,4,6-Triisopropylphenylsulfonyl-(L)-3-cyan-phenylalanin-4-ethoxycarbonyl-piperazid

5,7 g der Verbindung 1.2 wurden in 100 ml Tetrahydrofuran (THF) gelöst, auf 0°C abgekühlt, 2,22 g α-Hydroxybenzotriazol (HOBt), 2,82 g Dicyclohexylcarbodiimid (DCC) zugefügt und 30 min gerührt. Nach Zugabe von 3,94 g 1-Ethoxycarbonyl-piperazin in 30 ml THF wurde über Nacht gerührt, danach ausgefallenes Dicyclohexylurea (DCU) abfiltriert, das Lösungsmittel abdestilliert und das erhaltene Rohprodukt über KG 60 (Chloroform) gereinigt. Ausbeute: 7,1 g eines amorphen Pulvers (96%).

### 1.4 Nα-2,4,6-Triisopropylphenylsulfonyl-(L)-3-amidino-phenylalanin-4-ethoxycarbonyl-piperazid Hydrochlorid

7,1 g der Verbindung 1.3 wurden in 30 ml Pyridin gelöst, 30 Tropfen Triethanolamin (TEA) zugefügt, 10 min ein kräftiger Schwefelwasserstoffstrom eingeleitet und 2 Tage bei Raumtemperatur stehengelassen. Anschließend wurde das Lösungsmittel abdestilliert, der Rückstand in Ethylacetat gelöst, die organische Phase mit 1 N Salzsäure und gesättigter Kochsalzlösung gewaschen, über MgSO₄ getrocknet und das Lösungsmittel abdestilliert. 7,2 g des auf diese Weise erhaltenen Thioamids wurden in 250 ml Aceton gelöst, die Lösung mit 17 g Methyliodid versetzt und 2 Tage bei Raumtemperatur unter Lichtschutz stehengelassen. Danach wurde das Lösungsmittel abdestilliert, das Thioimidester-hydroiodid (8,5 g) in 50 ml Methanol gelöst, 1,9 g Ammoniumacetat zugefügt und der Ansatz 4 Std. bei 60°C erwärmt. Das nach Abdestillieren des Lösungsmittels erhaltene Rohprodukt wurde über Sephadex LH20 (Methanol) gereinigt. Das auf diese Weise erhaltene Amidin-hydroiodid wurde über lonenaustauscher (Amberlite IRA-420) in das Hydrochiorid übergeführt. Ausbeute: 5,3 g eines amorphen Pulvers (69%).

### 2. Nα-2,4,6-Triisopropylphenylsufonyl-(D,L)-3-amidinopenyl-alanylnipecotinsäure-benzylamid Hydrochlorid

### 2.1 Nα-2,4,6-Triisopropylphenylsulfonyl-(D,L)-3-cyan-phenylalanylnipecotinsäure-ethylester

4,56 g Na-2,4,6-Triisopropylphenylsulfonyl-(D,L)-3-cyan-phenylalanin (aus (D,L)-3-Cyanphenylalanin-methylester-hydrochlorid und dem entsprechenden Sulfochlorid analog 1.1 und 1.2 dargestellt), 1,5 g HOBt und 2,42 g DCC wurden in 50 ml DMF gelöst, 1 Std. gerührt und danach 2,36 g Nipecotinsäure-ethylester zugefügt. Nach Rühren über Nacht wurde ausgefallenes DCU abfiltriert, das Lösungsmittel abdestilliert, der Rückstand in wenig Methanol gelöst und zur Kristallisation stehengelassen. Der gebildete Niederschlag wurde abgesaugt, mit Methanol gewaschen und getrocknet. Ausbeute: 4,46 g (75%).

### 2.2 Nα-2,4,6-Triisopropylphenylsufonyl-(D,L)-3-cyan-phenylalanylnipecotinsäure

4,4 g des vorher beschriebenen Ethylesters wurden in einer Mischung aus 35 ml Essigsäure und 25 ml 1 N HCI 2 Std. unter Rückfluß erhitzt. Nach Zugabe von 10 ml Wasser wurde zum Erkalten stehengelassen, wobei sich ein wachsartiges Produkt abschied. Nach Abgießen des Lösungsmittels wurden 200 ml Wasser zugesetzt, längere Zeit kräftig gerührt und die erhaltene feste Substanz abgesaugt, mit Wasser gewaschen und getrocknet. Ausbeute: 3,84 g (92%).

### 2.3 Nα-2,4,6-Triisopropylphenylsulfonyl-(D,L)-3-cyan-phenylalanyl-nipecotinsäure-benzylamid

2,28 g der vorher beschriebenen Verbindung 0,6 g HOBt und 0,97 g DCC wurden in 20 ml DMF gelöst, 1 Std. gerührt, anschließend 0,6 g Benzylamin zugefügt und weiter über Nacht gerührt. Nach Abfiltrieren des ausgefallenen DCU wurde das Lösungsmittel abdestilliert, der Rückstand in Methanol gelöst und die Lösung in 5-proz. Natriumhydrogencarbonatlösung/Eis gegossen. Nach 1 Std. wurde der gebildete Niederschlag abgesaugt, mit Wasser gewaschen und im Vakuum getrocknet. Ausbeute: 2,48 g (94%).

### 2.4 Nα-2,4,6-Triisopropylphenylsulfonyl-(D,L)-3-amidino-phenylalanylnipecotlnsäure-benzylamid Hydrochlorid

2,4 g der Verbindung 2.3 wurden in 30 ml Pyridin gelöst, 30 Tropfen TEA zugefügt, in die Lösung 10 min Schwefelwasserstoff eingeleitet und der Ansatz 2 Tage bei Raumtemperatur stehengelassen. Anschließend wurde das Lösungsmittel abdestilliert, der Rückstand in Ethylacetat aufgenommen und mit 1 N HCI ausgeschüttelt. Nach Waschen der organischen Phase mit gesättigter Kochsalzlösung und Trocknen über Natriumsulfat wurde das Lösungsmittel abdestilliert. 2,38 g des auf diese Weise erhaltenen Thioamids wurden in 100 ml Aceton gelöst, die Lösung mit 6,5 g Methyliodid versetzt und 20 Std. bei Raumtemperatur unter Lichtschutz stehengelassen. Danach wurde das Lösungsmittel abdestilliert, das Thioimidonester-hydroiodid in 50 ml Methanol gelöst, 0,5 g Ammoniumacetat zugegeben und der Ansatz 4 Std. bei 60°C im Wasserbad erwärmt. Das nach Abdestillieren des Lösungsmittels erhaltene Rohprodukt konnte über KG 60 gereinigt werden. Die Elution erfolgte zunächst mit Chloroform, danach mit Chloroform/Methanol 9:1. Das so gereinigte Amidin-hydroiodid wurde über Ionenaustauscher (Amberlite IRA-420) in das Hydrochlorid übergeführt. Ausbeute: 1,45 g eines amorphen Pulvers (56%).

Die Charakterisierung der Verbindungen erfolgte massenspektrometrisch, eine Reinheitsprüfung erfolgte mittels DC und HPLC.

### 3. In vitro Hemmung von Urokinase durch ausgewählte Verbindungen der Formel I

| **Konfiguration** | **R**^{**1**} | **R**^{**2**} | **n** | **Ki, µmol/l** |
|---|---|---|---|---|
| L | -N N-COOC₂H₅ | TIPP | 0 | 0,41 |
| D,L | -N N-COOC₂H₅ | TIPP | 0 | 0,96 |
| Abkürzungen: TIPP - 2,4,6-Triisopropylphenyl | | | | |

### Bestimmung der Hemmwirkung

Zur Bestimmung der Inhibitoraktivität wurden 200 µl Tris-Puffer (0,05 mol/l, den Inhibitor enthaltend, 0,154 mol/l NaCl, 5% Ethanol pH 8,0), 25 µl Substrat (Pefachrome UK oder Bz-ßAla-Gly-Arg-pNA in H₂O; Pentapharm Ltd., Basel, Schweiz) und 50 µl sc-Urokinase (Ribosepharm GmbH, Haan, Deutschland) bei 25°C inkubiert. Nach 3 min wurde die Reaktion durch Zugabe von 25 µl Essigsäure (50%) unterbrochen und die Absorption bei 405 nm mittels Microplate Reader (MR 5000, Dynatech, Denkendorf, Deutschland) bestimmt. Die Kᵢ-Werte wurden nach Dixon durch lineare Regression mittels eines Computerprogramms ermittelt. Die Kᵢ-Werte sind das Mittel aus mindestens 3 Bestimmungen, die Standardabweichung lag unter 25%.

### 4. In vitro Hemmung verschiedener Serinproteasen vom Trypsin-Typ durch (Nα-(2,4,6-Triisopropyl-phenylsulfonyl)-(L)-3-amidino-phenylalanin-4-ethoxycarbonyl-piperazid (uPA-Inhibitor) und Nα-2-Naphthylsulfonyl-3-amidinophenylalanin-N'-methylpiperazid (Napththylsulfonyl-Derivat) als Vergleich

| | Ki [µmol/l] | |
|---|---|---|
| **Enzym** | **uPA-Inh.** | **Naphthylsulfonyl-Der.** |
| Urokinase | 0,41 | 150 |
| Plasmin | 0,39 | 55 |
| Sc-tPA | 4,9 | 430 |
| Thrombin | 0,49 | 0,036 |
| Faktor Xa | 1,7 | 30 |
| Faktor XIIa | 13 | > 1000 |
| Plasma-Kallikrein | 7,2 | 85 |
| Glandulär-Kallikrein | > 1000 | > 1000 |
| Trypsin | 0,037 | 1,3 |
| Tryptase | 6,3 | 33 |

Die Bestimmung der Hemmwirkung für die verwendeten Enzyme erfolgte nach dem in Beispiel 3 beschriebenen Prinzip.

Aus den oben angegebenen Werten ist ersichtlich, daß der erfindungsgemäße Urokinasehemmstoff uPA-Inhibitor überraschenderweise einen um mehr als den Faktor 10 kleineren Kᵢ-Wert für Urokinase als für Einzelketten tPA (Sc-tPA) aufweist. Somit eignen sich die erfindungsgemäßen Substanzen als selektive Urokinaseinhibitoren. Zum Vergleich ist die Inhibitoraktivität des Naphthylsulfonyl-Derivats angeführt, das eine signifikant geringere in vitro Anti-uPA-Aktivität aufweist.

### 5. Bestimmung der Zytotoxizität

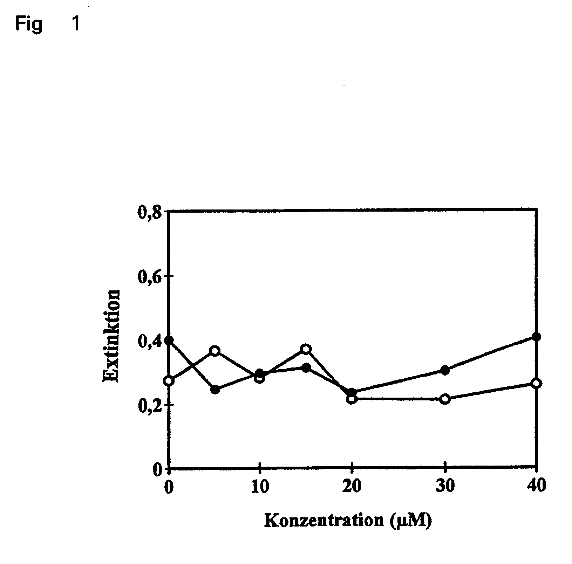

Für die Bestimmung der Zellproliferation/Zytotoxizität wurde ein kommerziell erhältlicher Testeingesetzt (Promega), welcher auf der zellulären Umsetzung eines Tetrazoliumsalzes beruht. Das aus dieser Reaktion resultierende farbige Produkt kann mittels eines ELISA-Spektrometers (ICN-Flow) quantifiziert werden. Der synthetische Inhibitor (offene Kreise) hatte gegenüber dem Lösungsmittel (geschlossene Kreise) alleine keinen Einfluß auf das Zellwachstum humaner Orvarialkarzinomzellen OV-MZ-6 (Figur 1). Somit ist der erfindungsgemäße uPA-lnhibitor in pharmakologisch wirksamen Konzentrationen bis 40 µM nicht zytotoxisch.

### 6. Inhibierung des Abbaus einer Fibrin-Matrix durch humane Mammakarzinomzellen

Zur Untersuchung der Fähigkeit von Tumorzellen eine extrazelluläre Matrix abzubauen, wurde ein Fibrinmatrixabbau-Test entwickelt und verwendet. Je größer die proteolytische Aktivität der Tumorzellen, um so höher ist die Konzentration der Fibrinabbauprodukte im Matrixüberstand. Der Matrixabbaukapazität entspricht der Konzentration der Fibrinabbauprodukte, welche mittels ELISA (D-Dimer) ermittelt werden.

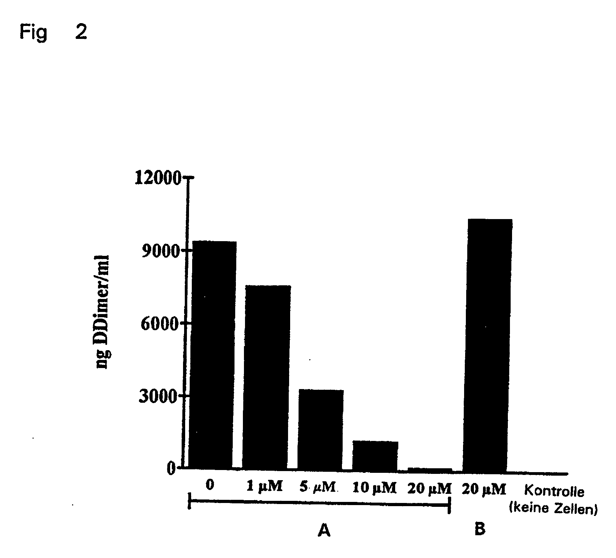

Die Fibringele wurden in 24 Well Kulturplatten aus 200 ml Fibrinogen (50 mg/ml) in PBS (pH 7,4) durch 50 µl Thrombin (10 U/ml) und 50 µl CaCl₂ (150 mM) pro Well nach Inkubation für 30 min bei 37°C gebildet. 2 x 10⁵ Mammakrzinomzellen wurden auf diese Fibrinmatrix in 1 ml DMEM Kulturmedium, plus 10% fetalem Kälberserum und 2 µg Glu-Plasminogen ausgesät und 4 h lang inkubiert. Danach wurde der Überstand zentrifugiert, um Zellen zu entfernen, und die Fibrinabbauprodukte mittels ELISA quantifiziert. Die Zugabe des erfindungsgemäßen Inhibitors (A) in unterschiedlichen Konzentrationen führte zu einer signifikanten Inhibierung des Matrixabbaus durch Mammakarzinomzellen im Vergleich zu dem Naphthyl-Derivat (B), das keine Hemmung des Fibrinabbaus durch Mammakarzinomzellen zeigt (Figur 2).

### 7. In vivo Test des uPA Inhibitors auf Tumorausbreitung, Tumorwachstum und Metastasierung in der Ratte

### A) Brustkrebs-Modell

10-25 mm³ Rattenbrustkrebs-Tumorfragmente BN-472 wurden subkutan orthotropisch unter die Fettpolster der Brustdrüse in 6 bis 7 Wochen alten, weiblichen Brown Norwegian Ratten transplantiert (Tag 0). Die Behandlung der Tiere wurde 24 h nach der Tumorinokulation intraperitoneal begonnen. Jede Gruppe bestand aus 8 Tieren. Die Kontrottgruppe erhielt nur injektionslösung (100 µl einer 10% Ethanol/Saline (0,9% NaCl) Lösung). Der Vergleichsgruppe des Naphthyl-Derivats (B) und der Therapiegruppe des erfindungsgemäßen uPA-Inhibitors (A) wurden in der oben genannten Lösung eine Dosis von 1 mg/kg Körpergewicht täglich i.p. verabreicht. Die Behandlung wurde über einen Zeitraum von 4 Wochen durchgeführt.

Die Abmessungen der subkutanen Tumoren und die Gewichte der Tiere wurden wöchentlich bestimmt. Am Ende der Behandlung wurden die Tiere getötet und Tumorgewichte, Organgewichte und die Anzahl von Metastasen in relevanten Geweben bestimmt.

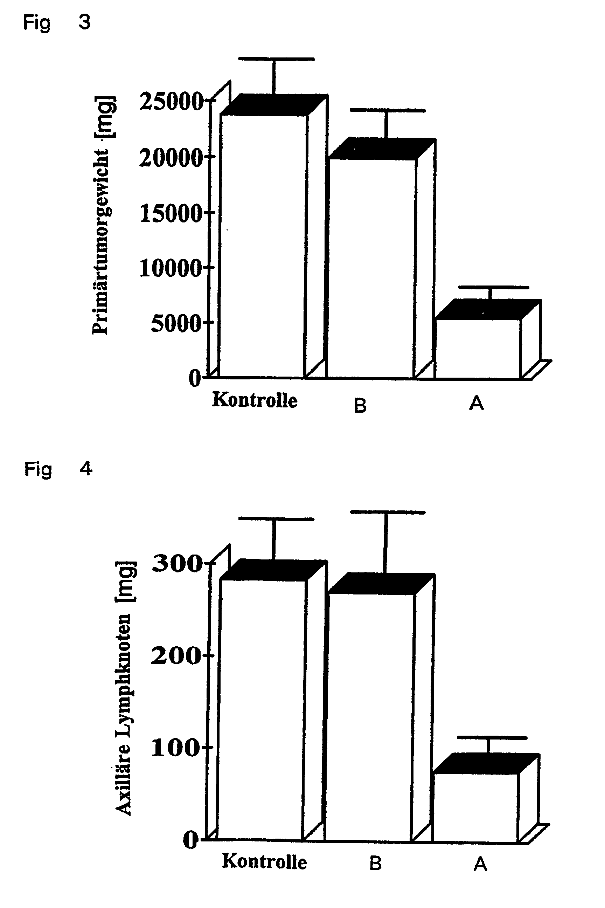

Die Behandlung mit uPA-lnhibitor (A) führte zu einer signifikanten Reduktion des Primärtumorgewichtes sowie der axillären Lymphknoten (p = 0,003 bzw. p = 0,005) im Vergleich mit dem Naphthyl-Derivat (B) und Kontrollgruppen ohne Inhibitor (Fig. 3 und 4). Die Gewichte von Lunge, Leber, Niere und Milz bei den mit dem uPA-lnhibitor behandelten Tieren waren unverändert gegenüber den Kontrolltieren.

### B) Pankreaskarzinom-Modell

Fragmente des transplantierbaren und metastasierenden Pankreasadenokarzinoms CA20948 der Ratte wurden aus Donortieren explantiert. Nach Zellvereinzelung wurden gleiche Mengen an suspendierten Tumorzellen zusammen mit 2 mg Matrigel jedem der Empfängertiere, männlichen 10 Wochen alten Lewisratten (n=9), subkutan implantiert. Die Durchführung der Behandlung sowie Zusammensetzung der Therapiegruppen war identisch wie unter A).

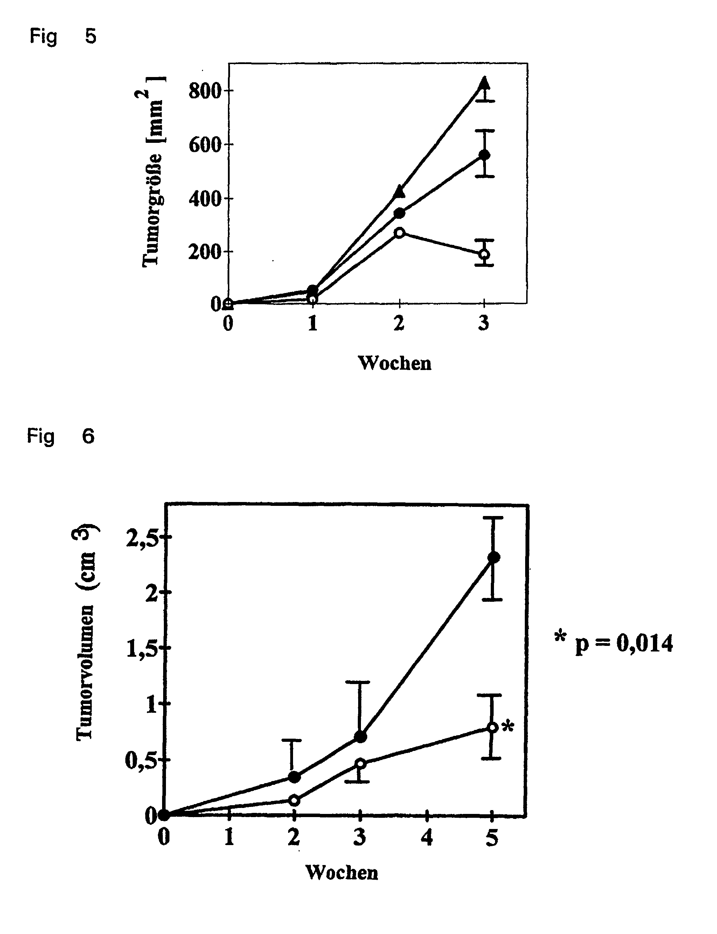

Wie aus Figur 5 ersichtlich ist, findet man beim erfindungsgemäßen uPA-Inhibitor (offene Kreise) gegenüber dem Naphthyl-Derivat (geschlossene Kreise) und der Kontrollgruppe (Dreiecke) eine signifikante Verringerung des Tumorgewichts und eine Verminderung des Wachstums bei den entstehenden Ratten-Pankreaskarzinom.

### C) Wiederholung der Versuche mit geänderter Applikationsart

Die in den Abschnitten A und B beschriebenen Versuche wurden mit geänderter Applikationsart des Inhibitors wiederholt. Dabei wurde ohne Veränderung der täglichen Dosis der Inhibitor im Mammakarzinom-Modell subkutan (n = 9) und im Pankreasadeonkarzinom-Model) intraperitonial (n = 8) appliziert. Die Ergebnisse dieser Wiederholungsversuche entsprachen in Tendenz und Umfang den bereits dargestellten Ergebnissen.

### D) Zusammenfassung der Ergebnisse

In allen Versuchen wurde durch Behandlung mit dem Inhibitor eine erhebliche Reduktion der Tumorgröße bzw. des Tumorgewichts und der Anzahl bzw. der Masse von Tochtergeschwülsten im Vergleich zu den Kontrollgruppen erreicht. Im Mammatumor-Modell waren in der Inhibitor-behandelten Gruppe, die mittleren Tumorgewichte am Ende der Behandlung auf 23% (i.p.) bzw. 37% (auf s.c.) im Vergleich zu der mit Vehikel behandelten Kontrolle reduziert. Die Anzahl der Lungenfoci in Inhibitor-behandelten Gruppen waren auf 9% (i.p.) bzw. 32% (s.c.) und die mittleren Gewichte der axillären Lymphknoten auf 27% (i.p.) bzw. 48% (s.c.) reduziert.

Im Pankreastumor-Modell waren die Massen des tumorhaltigen Pankreas in den Inhibitor-behandelten Gruppen um 76% (i.p.) bzw. 34% (s.c.), die Massen der subkutanen Tumoren um 54% (i.p.) bzw. 60% (s.c.) verglichen mit den jeweiligen Vehikel-behandelten Gruppen reduziert. Die Anzahl detektierter Leberfoci in Inhibitor-behandelten Gruppen war 29% (i.p.), bzw. 2% (s.c.) im Vergleich zu den Vehikel-behandelten Kontrollgruppen.

Die Entwicklung der Körpergewichtszunahme und der Vergleich der Organgewichte zwischen Inhibitor- und Vehikel-behandelten Gruppen ließen keine Hinweise für eine etwaige erhebliche Toxizität des Inhibitors unter den beschriebenen Bedingungen erkennen.

### 8. Behandlung humaner Brustkrebszellen in der Nacktmaus

Um die in vivo Effizienz des Inhibitors zur Hemmung des Tumorwachstums humaner Mammakarzinomzellen zu testen (MDA-BA-231), wurden 6 x 10⁶ Zellen subcutan in die rechte Flanke von Balb/c Nacktmäusen (4-6 Wochen alt) injiziert. Die Tumorzellen wurden vor Inokulation mit dem synthetischen uPA-lnhibitor vorinkubiert. Nach 24 h wurden die Mäuse mit einer Dosis von 1,2 mg/kg Körpergewicht intraperitoneal wie unter A) beschrieben zweimal wöchentlich behandelt. Die Tumorgröße wurde wöchentlich durch Messung der zwei größten Durchmesser bestimmt.

Wie aus Figur 6 ersichtlich ist, nimmt das Tumorvolumen bei der Verabreichung des uPA-Inhibitors (offene Kreise) signifikant langsamer zu als in der Kontrollgruppe (geschlossene Kreise) wo Ethanol in Saline verabreicht wurde.

### 9. Die Biodistribution des Inhibitors in der Ratte

Die Biodistribution des Inhibitors wurde in zwei unabhängigen Versuchen in Gewebeaufschlüssen von Ratten ermittelt, die 5 bzw. 10 Tage lang täglich einmal mit 1 mg/kg Inhibitor i.p. behandelt worden waren. Zum Aufschluß wurden jeweils 100 mg Gewebe mechanisch zerkleinert und mit 200 µl 1 % Triton X-100 in physiologischer Kochsalzlösung vermischt. Nach Zugabe von 400 µl Ethanol wurde der Aufschluß 1 min mit Ultraschall behandlet. Der Gewebeextrakt wurde 15 min bei 12.000 x g zentrifugiert. Zur Vorreinigung wurde der Überstand auf eine mit 1 ml Methanol und 1 ml Wasser equilibrierte C18-Silica Reversed Phase Vorsäule (Sep-Pak®cartridge C18, 1 ml Waters, Eschborn, Deutschland) aufgetragen, hintereinander mit 2 x 1 ml H₂O, 1 ml 10% Methanol, 1 ml H₂O, 1 ml 5% Acetonitril, 0,04% Perchlorsäure und 1 ml H₂O gewaschen und mit 500 µl 75 % Acetonitril, 0,04% Perchlorsäure eluiert. Die HPLC-Analytik wurde auf einer Reversed Phase C18 Silica Säule mit einem 5-55%igen Acetonitril Gradienten mit 0,04% Perchlorsäure durchgeführt.

Die Konzentrationen des Inhibitors in den jeweils untersuchten Gewebetypen (µg/g) sowie in Blutplasma und Galle (µg/ml) und zum Vergleich eines entsprechenden Naphthylsulfonylderivats sind in der nachfoigenden Tabelle dargestellt.

**Tabelle**

| Verteilungsprofil einer erfindungsgemäßen Substanz in verschiedenen Geweben bzw. Blutplasma und Galle im Vergleich zu einem Naphthylsulfonyl-Derivat (Nα-2-Naphthylsulfonyl-3-amidinophenylalanin-4-ethoxycarbonylpiperazid) | | | |
|---|---|---|---|
| | Gehalt (µg/g) | | |
| Gewebe | uPA-Inhibitor 1 mg/kg i.p. 5 Tage | uPA-Inhibitor 1 mg/kg i.p. 10 Tage | Naphthylsulfonyl-Drivat 1 mg/kg i.p., 5 Tage |
| Milz | 2,02 | 2,48 | 0,089 |
| Leber | 2,85 | 2,08 | 0,12 |
| Niere | 2,67 | 2,48 | 0,085 |
| Muskel | 0,74 | < 0,5 | 0,008 |
| Fett Niere | 0,82 | 1,0 | <0.005 |
| Herz | <0,5 | 1,09 | 0,59 |
| Lunge | 3,70 | 1,81 | 0,020 |
| Gehirn | <0,5 | <0,5 | |
| Lymphknoten Trachea | 7,45 | 16,38 | 0,12 |
| Lymphknoten Achsel | 1,97 | 2,74 | <0,005 |
| Lymphknoten Knie | 7,83 | 3,8 | <0,005 |

| | Gehalt (µg/ml) | | |
|---|---|---|---|
| Plasma | 0,008 | 0,035 | 0,004 |
| Galle | 1,96 | 1,75 | 0,097 |

In den meisten untersuchten Gewebetypen lag nach 5 bis 10 Tagen der Inhibitor in einer Größenordnung von 1 bis 3 µg/g vor. Die Plasmakonzentrationen des Inhibitors lagen 24 h nach der jeweils letzten i.p. Applikation jeweils ein bis zwei Größenordnungen unter den mittleren Gewebekonzentrationen. Daraus kann auf eine hohe Affinität für Gewebe und eine niedrige Plasmaeiweißbindung geschlossen werden. Die Konzentrationen des im Vergleich über 5 Tage applizierten Naphthylsulfonyl-Derivats waren in den verschiedenen Geweben 20-30 fach geringer.

Der Inhibitor zeigt eine auffallends Anreicherung in den Lymphknoten. In den unabhängigen Versuchen wurden in trachealen Lymphknoten nach 5-tägiger Applikation Konzentrationen 5,3 bzw. 7,5 µg/g, nach 10 tägiger Applikation 21,6 bzw. 16,4µg/g gemessen. Da Tumorzellen sich regelmäßig über Iymphatische Bahnen disseminieren, ist die spezifische Anreicherung des Inhibitors in den Lymphgefäßen von Bedeutung und Vorteil für dessen Einsatz als anti-metastatisches Therapeutikum.

## Patentansprüche

1. Verwendung von Verbindungen der Formel I 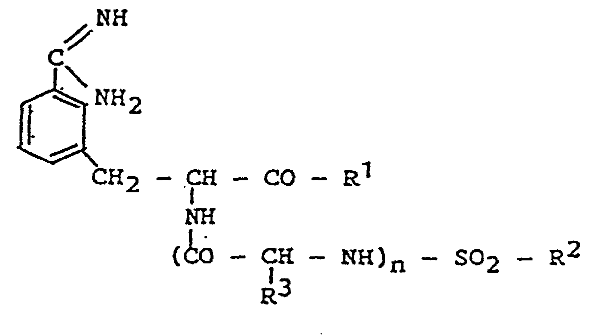 die als Racemate sowie als D- oder L-konfigurierte Verbindungen vorliegen und in denen
R1
(a) OH oder OR⁴ ist, wobei R⁴ ein gegebenenfalls substituiertes, verzweigtes oder unverzweigtes C₁-C₈-Alkyl, C₃-C₈-Cycloalkyl oder Aralkyl ist,
(b) eine Gruppe der Formel 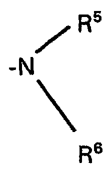 darstellt, in welcher R⁵ und R⁶ beliebige Reste sind, wobei insbesondere
(i) R⁵ and R⁶ H sind,
(ii) R⁵ H ist und R⁶ ein gegebenenfalls substituiertes verzweigtes oder unverzweigtes C₁-C₈-Alkyl, Aralkyl oder C₅-C₈ Cycloalkyl ist,
(iii) R⁵ und R⁶ jeweils unabhängig ein gegebenenfalls substituiertes, unverzweigtes oder verzweigtes C₁-C₄ Alkyl sind oder
(iv) R⁵ H ist und R⁶ -NH₂ oder eine insbesondere mit Aryl oder Heteroaryl substituierte Aminogruppe ist,
(v) R⁵ H oder ein gegebenenfalls substituiertes, unverzweigtes oder verzweigtes C₁-C₄ Alkyl ist oder R⁶ der Rest einer Aminosäure, eines Peptids oder eines Polypeptids ist,
(c) eine Gruppe der Formel 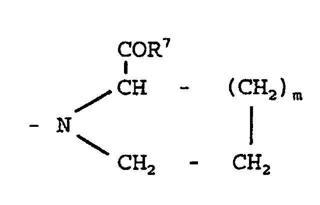 darstellt, in welcher m die Zahl 1 oder 2 bezeichnet, und in welcher eine oder mehrere der Methylengruppen gegebenenfalls substituiert sind, wobei die Gruppe (c) racemisch oder D- bzw. L-konfiguriert ist, und R⁷ die Bedeutung von R¹ in den Ziffern (a), (b) und (f) aufweist,
(d) eine Gruppe der Formel 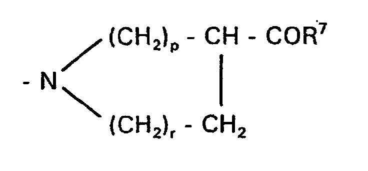 darstellt, in welcher p = r = 1, p = 1 und r = 2 oder p = 2 und r = 1 sind und in welcher eine oder mehrere der Methylengruppen gegebenenfalls substitutiert sind, und R⁷ die Bedeutung von R¹ in Ziffer (a), (b) und (f) aufweist.
(e) eine Piperidylgruppe darstellt, die gegebenenfalls in einer der Stellungen 2, 3 und 4 substituiert ist,
wobei gegebenenfalls an die heterocycloaliphatischen Ringe der Formeln (c), (d), (e) ein weiterer aromatischer oder cycloaliphatischer Ring in 2,3 oder 3,4 Stellung, bezogen auf das Heteroatom, ankondensiert ist,
(f) eine Gruppe der Formel 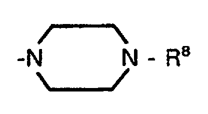 darstellt, in welcher R⁸
(i) einen gegebenenfalls substituierten C₁-C₆-Alkyl- oder Arylrest,
(ii) einen gesättigten oder ungesättigten, verzweigten oder unverzweigten C₁-C₆-Alkoxyrest oder
(iii) einen gegebenenfalls substituierten Phenoxy- bzw. Benzyloxycarbonylrest bedeutet,
(g) einen Acylrest der Formel -COX darstellt, wobei X
(i) H, einen gegebenenfalls substituierten unverzweigten oder verzweigten Alkylrest,
(ii) einen gegebenenfalls substituierten Aryl- oder Heteroarylrest, oder
(iii) einen gegebenenfalls substituierten Cycloalkylrest bedeutet,
(h) einen Aralkylrest darstellt, in dem der aromatische Rest gegebenenfalls substituiert ist,
(i) einen Carbonsäureamidrest der Formel -CONR'R", einen Thiocarbonsäureamidrest -CSNR'R" oder einen Essigsäureamidrest -CH₂-CONR'R" darstellt, wobei
(i) R' und R" H sind,
(ii) R' und R" jeweils unabhängig C₁-C₄-Alkyl sind,
(iii) R' H ist und R" C₁-C₄-Alkyl ist,
(iv) R' H ist und R" Aryl ist, oder
(v) R' und R" mit dem Stickstoffatom einen heterocycloaliphatischen Ring mit 5-7 Ringgliedern, der ein weiteres Heteroatom tragen kann, bilden,
(j) einen SO₂-Y-Rest darstellt, in dem Y
(i) ein gegebenenfalls substitutiertes C₁-C₈-Alkyl,
(ii) ein gegebenenfalls substituiertes Aryl oder Heteroaryl bzw. O-Aryl oder O-Heteroaryl, oder
(iii) -NR'R" ist, wobei R' und R" jeweils unabhängig H oder C₁-C₃-Alkyl bedeuten,
(k) einen cycloaliphatischen Ring mit 5 bis 8 C-Atomen darstellt, der gegebenenfalls substituiert ist,
(I) einen gegebenenfalls substituierten Heteroarylrest oder heterocycloaliphatischen Rest darstellt,
(m) einen funktionalisierten Alkylrest der Formel -(CH₂)ₙ-X darstellt, wobei die Alkylkette unverzweigt oder verzweigt ist, n = 1 bis 8 bedeutet und der funktionelle Rest X
(i) eine Hydroxylgruppe darstellt, deren H-Atom gegebenenfalls durch eine C₁-C₄-Alkyl-, Aralkyl-, z.B. Benzyl oder Phenylethyl, Aryl-, C₁-C₄-Hydroxyalkyl- oder Acylgruppe CO-Alkyl (C₁-C₆), substituiert ist,
(ii) ein Halogenatom bedeutet,
(iii) eine tertiäre Aminogruppe der Formel -N(Alk)₂ darstellt, wobei die Alkylgruppen 1 bis 3 C-Atome besitzen und das Stickstoffatom gegebenenfalls einem heterocycloaliphatischen Ring mit 5-7 Ringgliedern, der ein weiteres Heteroatom, S tragen kann, angehört,
R² einen gegebenenfalls substituierten Phenylrest darstellt,
R³ H oder verzweigtes bzw. unverzweigtes C₁-C₄-Alkyl ist und n 0 oder 1 bedeutet.
oder von Salzen der Verbindungen zur Herstellung eines Mittels für die Diagnostik, Therapie und Prävention von Urokinase- oder Urokinaserezeptor-assoziierten Krankheiten.

2. Verwendung nach Anspruch 1,
**dadurch gekennzeichnet,**
**dass** R¹ eine Gruppe der Formeln (b), (d) und (f) ist, R² einen 2,4,6-Triisopropylphenyl-Rest darstellt, und n=0 ist.

3. Verwendung nach Anspruche 1 oder 2,
**dadurch gekennzeichnet,**
**dass** die Verbindung der Formel I Nα-(2,4,6-Triisopropyl-phenylsulfonyl)-3-amidino-(D,L)-phenylalanin-4-ethoxycarbonyl-piperazid, das L-Enantiomer oder ein pharmazeutisch verträgliches Salz einer der Verbindungen ist.

4. Verwendung nach einem der Ansprüche 1 bis 3,
**dadurch gekennzeichnet,**
**dass** die Verbindungen in Form von physiofogisch verträglichen Säuresalzen, insbesondere als Hydrochloride, vorliegen.

5. Verwendung nach einem der Ansprüche 1 bis 4 zur Tumorbekämpfung.

6. Verwendung nach Anspruch 5 zur Bekämpfung von Mammakarzinomen, Pankreaskarzinomen und der Metastasenbildung.

7. Verwendung nach einem der Ansprüche 1 bis 4 zur Bekämpfung von Pemphigus vulgaris.

8. Verwendung nach einem der Ansprüche 1 bis 7,
**dadurch gekennzeichnet,**
**dass** die Verbindungen der Formel I als Konjugate mit weiteren pharmakologisch wirksamen Substanzen eingesetzt werden.

9. Verwendung nach Anspruch 8,
**dadurch gekennzeichnet,**
**dass** die Verbindungen als Konjugate mit Radiomarkierungen oder mit zytotoxischen Substanzen eingesetzt werden.

10. Verwendung nach einem der Ansprüche 1 bis 9 zur Herstellung von oral, topisch, rektal oder parenteral verabreichbaren Arzneimitteln.

11. Verwendung nach einem der Ansprüche 1 bis 10 in Form von Tabletten, Dragees, Kapseln, Pellets, Suppositorien, Lösungen oder transdermalen Systemen, wie Pflastern.

## Claims

1. The use of compounds of the formula I 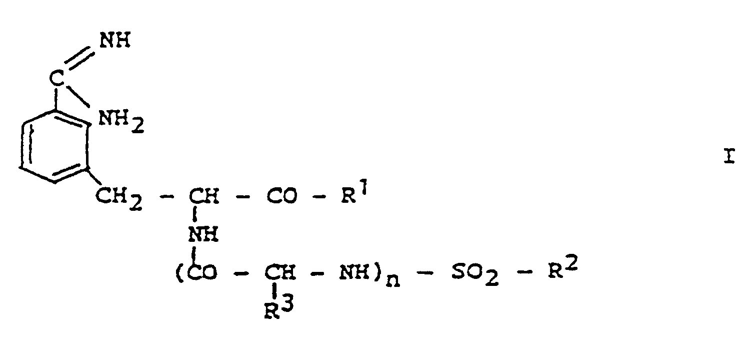 which are present as racemates and also as D- or L-configured compounds and in which
R¹
(a) is OH or OR⁴, where R⁴ is unsubstituted or substituted, branched or unbranched C₁-C₈-alkyl, C₃-C₈-cycloalkyl or aralkyl,
(b) represents a group of the formula 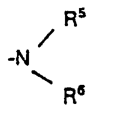 in which R⁵ and R⁶ are any radicals, where in particular
(i) R⁵ and R⁶ are H,
(ii) R⁵ is H and R⁶ is unsubstituted or substituted, branched or unbranched C₁-C₈-alkyl, aralkyl or C₅-C₈-cycloalkyl,
(iii) R⁵ and R⁶ are in each case independently unsubstituted or substituted, branched or unbranched C₁-C₄-alkyl or
(iv) R⁵ is H and R⁶ is -NH₂ or is, in particular, an aryl-substituted or heteroaryl-substituted amino group,
(v) R⁵ is H or unsubstituted or substituted, branched or unbranched C₁-C₄-alkyl or R⁵ is an amino acid residue, a peptide residue or a polypeptide residue,
(c) represents a group of the formula 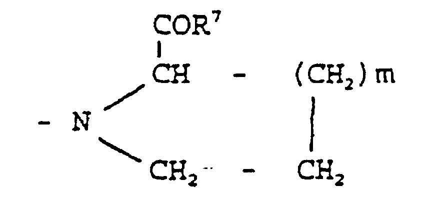 in which m is the number 1 or 2 and in which one or more of the methylene groups are unsubstituted or substituted, with the group (c) being racemic or in D or L configuration, and R⁷ has the meaning of R¹ in subsections (a), (b) and (f),
(d) represents a group of the formula 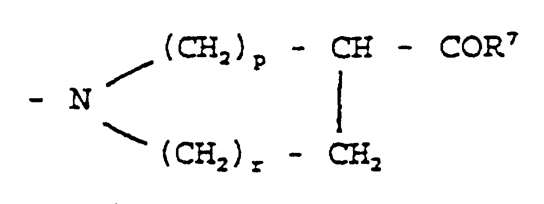 in which p = r = 1, p = 1 and r = 2 or p = 2 and r = 1 and in which one or more of the methylene groups are unsubstituted or substituted and R⁷ has the meaning of R¹ in subsections (a), (b) and (f),
(e) represents a piperidyl group which is unsubstituted or substituted in one of positions 2, 3 or 4,
where a further aromatic or cycloaliphatic ring is optionally fused to the heterocycloaliphatic rings of the formulae (c), (d) and (e) in the 2,3 position or the 3,4 position relative to the heteroatom,
(f) represents a group of the formula 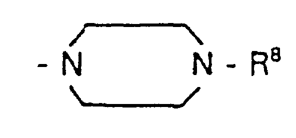 in which R⁸ is
(i) unsubstituted or substituted C₁-C₆-alkyl or aryl,
(ii) saturated or unsaturated, unbranched or branched C₁-C₆-alkoxy or
(iii)unsubstituted or substituted phenoxy or benzyloxycarbonyl,
(g) represents an acyl radical of the formula -COX, where X is
(i) H, unsubstituted or substituted, unbranched or branched alkyl
(ii) unsubstituted or substituted aryl or heteroaryl, or
(iii)unsubstituted or substituted cycloalkyl,
(h) represents aralkyl in which the aromatic radical is unsubstituted or substituted,
(i) represents a carboxamide radical of the formula -CONR'R", a thiocarboxamide radical, -CSNR'R" or an acetamide radical -CH₂-CONR'R" where
(i) R' and R" are H,
(ii) R' and R" are in each case independently C₁-C₄-alkyl,
(iii) R' is H and R" is C₁-C₄-alkyl,
(iv) R' is H and R" is aryl, or
(v) R' and R" constitute together with the nitrogen atom a heterocycloaliphatic ring having 5-7 ring members and possibly having a further heteroatom,
(j) represents SO₂-Y where Y is
(i) unsubstituted or substituted C₁-C₈-alkyl,
(ii) unsubstituted or substituted aryl or heteroaryl or O-aryl or O-heteroaryl or
(iii) -NR'R", where R' and R" are in each case independently H or C₁-C₃-alkyl,
(k) represents a cycloaliphatic unsubstituted or substituted ring having from 5 to 8 carbon atoms,
(l) represents an unsubstituted or substituted heteroaryl or heterocycloaliphatic radical,
(m) represents a functionalized alkyl radical of the formal -(CH₂)ₙ-X, where the alkyl chain is unbranched or branched, n = 1 to 8, and the functional radical X
(i) represents a hydroxyl group whose hydrogen atom is unsubstituted or substituted by C₁-C₄-alkyl-, aralkyl-, e.g. benzyl or phenylethyl, aryl, C₁-C₄-hydroxyalkyl or acyl group CO-alkyl (C₁-C₆),
(ii) is a halogen atom
(iii) represents a tertiary amino group of the formula -N(alk)₂, where the alkyl groups have 1 to 3 carbon atoms and the nitrogen atom may belong to a heterocycloaliphatic ring having 5-7 ring members and possibly having a further heteroatom, S,
R² represents unsubstituted or substituted phenyl,
R³ is H or branched or unbranched C₁-C₄-alkyl, and n is 0 or 1,
or of salts of said compounds for preparing an agent for the diagnosis, therapy and prevention of urokinase-associated or urckinase receptor-associated disorders.

2. The use as claimed in claim 1,
**characterized in that**
R¹ is a group of the formulae (b), (d) and (f), R² represents 2,4,6 triisopropylphenyl, and n = 0.

3. The use as claimed in claim 1 or 2,
**characterized in that**
the compound of the formula I is Nα-(2,4,6-triisopropylphenylsulfonyl)-3-amidino-(D,L)-phenylalanine 4-ethoxycarbonylpiperazide, is the L enantiomer or a pharmaceutically suitable salt of one of the compounds.

4. The use as claimed in any of clams 1 to 3,
**characterized in that**
the compounds are present in the form of physiologically acceptable acid salts, in particular as hydrochlorides.

5. The use as claimed in any of claims 1 to 9 for controlling tumors.

6. The use as claimed in claim 5 for controlling breast carcinomas, pancreatic carcinomas and the formation of metastases.

7. The use as claimed in any of claims 1 to 4 for controlling pemphigus vulgaris.

8. The use as claimed in any of claims 1 to 7,
**characterized in that**
the compounds of the formula I are used coupled with further pharmacologically active substances.

9. The use as claimed in claim 8,
**characterized in that**
the compounds are used coupled with radiolabels or with cytotoxic substances.

10. The use as claimed in any of claims 1 to 9 for preparing medicaments which are administrable orally, topically, rectally or parenterally.

11. The use as claimed in any of claims 1 to 10 in the form of tablets, coated tablets, capsules, pellets, suppositories, solutions or transdermal systems such as plasters.

12. Nα(2,4,6-Triisopropylphenylsulfonyl)-3-amidino-(D,L)-phenylalanine 4-ethoxycarbonylpiperazide, the L enantiomer thereof or a pharmaceutically suitable salt of one of the compounds.

## Revendications

1. Utilisation de composés de formule I 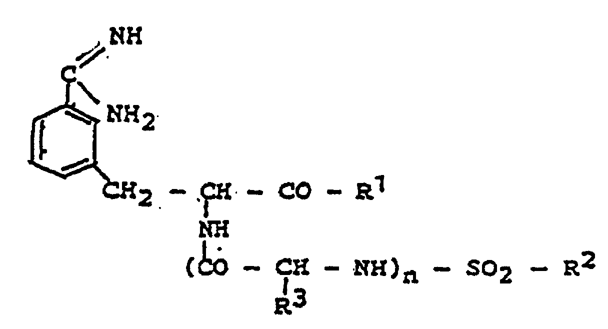 sous forme de racémates ou comme composés à configuration en D- ou en L- et dans lesquels
R¹
(a) est OH ou OR⁴, où R⁴ est un alkyle en C₁ à C₈, un cycloalkyle en C₃ à C₈ ou un aralkyle ramifié ou non ramifié, éventuellement substitué,
(b) représente un groupe de formule 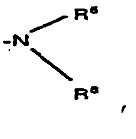 dans laquelle
R⁵ et R⁶ sont des résidus quelconques, où en particulier
(i) R⁵ et R⁶ sont H,
(ii) R⁵ est H et R⁶ est un alkyle en C₁ à C₈, un aralkyle ou un cycloalkyle en C₅ à C₈ ramifié ou non ramifié, éventuellement substitué,
(iii) R⁵ et R⁶ sont indépendamment un alkyle en C₁ à C₄, non ramifié ou ramifié, éventuellement substitué ou
(iv) R⁵ est H et R⁶ est -NH₂ ou un groupe amino substitué particulièrement avec un aryle ou un hétéroaryle,
(v) R⁵ est H ou un alkyle en C₁ à C₄ non ramifié ou ramifié éventuellement substitué ou R⁶ est un résidu d'acide aminé, de peptide ou de polypeptide,
(c) représente un groupe de formule 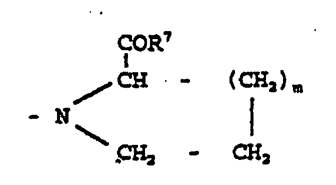 dans laquelle m est 1 ou 2 et dans laquelle un ou plusieurs des groupes méthyle sont substitués le cas échéant, où le groupe (c) est racémique ou configuré en forme D ou L, et R⁷ a la signification de R¹ dans les paragraphes (a), (b) et (f),
(d) représente un groupe de formule 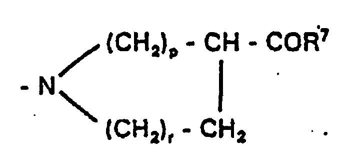 dans laquelle p = r = 1, p = 1 et r = 2, ou p = 2 et r = 1 et dans laquelle un ou plusieurs des groupes méthyle sont éventuellement substitués, et R⁷ a la signification de R¹ dans les paragraphes (a), (b) et (f).
(e) représente un groupe pipéridyle éventuellement substitué dans l'une des positions 2, 3 et 4 dans lequel un autre cycle aromatique ou cycloaliphatique est condensé aux cycles hétérocycloaliphatiques de formules (c), (d), (e) dans la position 2,3 ou 3,4 par rapport à l'hétéroatome.
(f) représente un groupe de formule 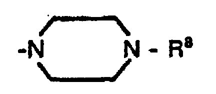 dans laquelle R⁸ représente
(i) un résidu alkyle en C₁ à C₆ ou aryle éventuellement substitué,
(ii) un résidu alcoxy en C₁ à C₆ saturé ou insaturé, ramifié ou non ramifié ou
(iii) un résidu phénoxycarbonyle ou benzyloxycarbonyle éventuellement substitué,
(g) représente un résidu acyle de formule -COX, dans laquelle X signifie
(i) H, un résidu alkyle non ramifié ou ramifié, éventuellement substitué,
(ii) un résidu aryle ou hétéroaryle éventuellement substitué, ou
(iii) un résidu cycloalkyle éventuellement substitué,
(h) représente un résidu aralkyle, dans lequel le résidu aromatique est substitué le cas échéant,
(i) représente un résidu carbonylamide de formule -CONR'R", un résidu thiocarbonylamide -CSNR'R" ou un résidu éthylamide -CH2-CONR'R", où
(i) R' et R" sont H,
(ii) R' et R" sont chacun indépendamment un alkyle en C₁ à C₄,
(iii) R' est H et R" est un alkyle en C₁ à C₄,
(iv) R' est H et R" est un aryle, ou
(v) R' et R" forment avec l'atome d'azote un cycle hétérocycloaliphatique de 5 à 7 éléments, qui peut porter un hétéroatome supplémentaire,
(j) représente un résidu SO₂-Y dans lequel Y
(i) est un alkyle en C₁ à C₈ éventuellement substitué,
(ii) un aryle ou hétéroaryle, notamment un O-aryle ou O-hétéroaryle éventuellement substitué, ou
(iii) -NR'R", où R' et R" sont indépendamment H ou un alkyle en C₁ à C₃,
(k) représente un cycle cycloaliphatique éventuellement substitué de 5 à 8 atomes de carbone,
(l) un résidu hétéroaryle ou un résidu hétérocycloaliphatique éventuellement substitué,
(m) représente un résidu alkyle fonctionnalisé de formule -(CH₂)ₙ-X, où la chaîne alkyle est non ramifiée ou ramifiée, n = 1 à 8, et le résidu fonctionnel X
(i) représente un groupe hydroxyle, dont l'atome H est substitué le cas échéant avec un groupe alkyle en C₁ à C₄ alkyle, un groupe aralkyle, par exemple le benzyle ou le phényléthyle, un groupe aryle, un groupe hydroxyalkyle en C₁ à C₄, ou un groupe acyle CO-alkyle en C₁ à C₆,
(ii) signifie atome d'halogène,
(iii) représente un groupe amino tertiaire de formule -N(Alk)₂, où les groupes alkyle comportent 1 à 3 atomes de carbone et l'atome d'azote peut appartenir à un cycle hétérocycloaliphatique de 5 à 7 éléments, qui peut porter un hétéroatome supplémentaire S,
R² représente un résidu phényle substitué le cas échéant,
R³ représente H ou un alkyle en C₁ à C₄ ramifié ou non ramifié, et n est égal à 0 ou 1
ou des sels de ces composés pour la fabrication d'un moyen de diagnostic, de thérapie et de prévention de maladies associées à l'urokinase ou au récepteur d'urokinase.

2. Utilisation selon la revendication 1, **caractérisée en ce que** R¹ est un groupe de formule (b), (d) et (f), R² est un résidu 2,4,6-triisopropylphényle, et n = 0.

3. Utilisation selon la revendication 1 ou 2, **caractérisée en ce que** le composé de formule I Nα-(2,4,6-triisopropyl-phényl-sulfonyl)-3-amidino-(D,L)-phénylalanin-4-éthoxycarbonyl-pipérazide, son énantiomère L ou un sel pharmaceutiquement acceptable constitue l'un des composés.

4. Utilisation selon l'une quelconque des revendications 1 à 3, **caractérisée en ce que** les composés sont sous la forme de sels d'acide acceptables d'un point de vue physiologique, en particulier de chlorhydrates.

5. Utilisation selon l'une quelconque des revendications 1 à 4 pour le traitement de tumeurs.

6. Utilisation selon la revendication 5 pour le traitement de carcinomes des seins, de carcinomes du pancréas, et du développement de métastases.

7. Utilisation selon l'une quelconque des revendications 1 à 4 pour le traitement de Pemphigus vulgaris.

8. Utilisation selon l'une quelconque des revendications 1 à 7, **caractérisée en ce que** les composés de formule I sont conjugués avec d'autres substances actives d'un point de vue pharmacologique.

9. Utilisation selon la revendication 8, **caractérisée en ce que** les composés sont conjugués avec des radiomarqueurs ou substances cytotoxiques.

10. Utilisation selon l'une quelconque des revendications 1 à 9 pour la préparation de médicaments pouvant être administrés par voie buccale, topique, rectale ou parentérale.

11. Utilisation selon l'une quelconque des revendications 1 à 10 sous forme de comprimés, dragées, capsules, pastilles, suppositoires, solutions ou systèmes transdermiques tels que des patchs.

12. Nα-(2,4,6-triisopropyl-phénylsulfonyl)-3-amidino-(D,L)-phénylalanin-4-éthoxycarboxyl-pipérazide, son énantiomère L ou un sel de l'un des composés acceptable d'un point de vue pharmaceutique.
